(19) Europäisches Patentamt
European Patent Office
Office européen des brevets

(11) Veröffentlichungsnummer: **0 612 719 A1**

# (12) EUROPÄISCHE PATENTANMELDUNG

(21) Anmeldenummer: **94250072.9**

(22) Anmeldetag: **29.01.90**

(51) Int. Cl.[5]: **C07C 235/06**, C07D 295/18, A61K 31/16, A61K 31/40, A61K 31/535

Diese Anmeldung ist am 23 - 03 - 1994 als Teilanmeldung zu der unter INID-Kode 60 erwähnten Anmeldung eingereicht worden.

(30) Priorität: **17.02.89 DE 3905325**

(43) Veröffentlichungstag der Anmeldung: **31.08.94 Patentblatt 94/35**

(60) Veröffentlichungsnummer der früheren Anmeldung nach Art. 76 EPÜ: **0 413 006**

(84) Benannte Vertragsstaaten: **AT BE CH DE FR GB IT LI LU NL SE**

(71) Anmelder: **SCHERING AKTIENGESELLSCHAFT**
**Müllerstrasse 170/178**
**D-13353 Berlin (DE)**

(72) Erfinder: **Earnshaw, Christopher, Dr.**
**16 The Green**
**Chesterton, Cambridge CB4 1RP (GB)**
Erfinder: **Kirsch, Gerald, Dr.**
**Luciusstrasse 6 b**
**D-14199 Berlin (DE)**
Erfinder: **Rach, Petra**
**Antwerpener Strasse 1**
**D-13353 Berlin (DE)**
Erfinder: **Thieroff-Ekerdt, Ruth, Dr.**
**Am Vierrutenberg 47**
**D-13469 Berlin (DE)**
Erfinder: **Töpert, Michael, Dr.**
**Sigismundkorso 58**
**D-13465 Berlin (DE)**

## (54) Hydroxyalkancarbonsäure-Derivate.

(57) Die Erfindung betrifft Hydroxycarbonsäure-Derivate der allgemeinen Formel I

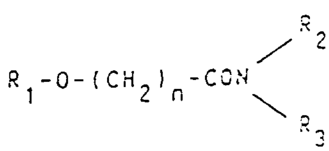

(I),

worin

| | |
|---|---|
| $R_1$ | ein Wasserstoffatom oder eine Acylgruppe mit maximal 16 Kohlenstoffatomen darstellt |
| $R_2$ und $R_3$ | jeweils Alkylgruppen mit maximal 8 Kohlenstoffatomen oder gemeinsam eine gegebenenfalls durch ein Sauerstoffatom oder ein Stickstoffatom unterbrochene Alkylengruppe mit 4 bis 8 Kohlenstoffatomen bedeutet und |
| n | die Ziffern 7 bis 18 symbolisiert, mit der Maßgabe, daß es nicht die Ziffer 10 bedeutet, wann $R_2$ und $R_3$ jeweils eine Ethylgruppe oder jeweils eine n-Butylgruppe darstellt und daß es nicht die Ziffer 15 bedeutet, wenn $R_2$ und $R_3$ jeweils eine Methylgruppe darstellt. |

EP 0 612 719 A1

Die Erfindung betrifft Hydroxyalkancarbonsäure-Derivate der allgemeinen Formel I

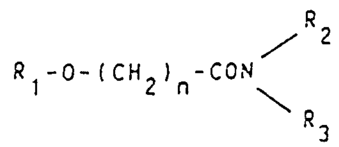 (I),

worin

| | |
|---|---|
| $R_1$ | ein Wasserstoffatom oder eine Acylgruppe mit maximal 16 Kohlensoffatomen darstellt |
| $R_2$ und $R_3$ | jeweils Alkylgruppen mit maximal 8 Kohlenstoffatomen oder gemeinsam eine gegebenenfalls durch ein Sauerstoffatom oder ein Stickstoffatom unterbrochene Alkylengruppe mit 4 bis 8 Kohlenstoffatomen bedeutet und |
| n | die Ziffern 7 bis 18 symbolisiert, mit der Maßgabe, daß es nicht die Ziffer 10 bedeutet, wenn $R_2$ und $R_3$ jeweils eine Ethylgruppe oder jeweils eine n-Butylgruppe darstellt und daß es nicht die Ziffer 15 bedeutet, wenn $R_2$ und $R_3$ jeweils eine Methylgruppe darstellt. |

In der Publikation von G. Czichocki et. al (Tenside Detergens 11, 1974 p 298-305) sind das 11-Hydroxyundecansäure-diethylamid und das 11-Hydroxyundecansäuredibutylamid vorbeschrieben und in der Publikation von D.F. Jones et. al (J. Chem.Soc.(C), 1968, p 2821-2827) wird das 16-Hydroxyhexadecansäure-dimethylamid erwähnt. Hinweise über die pharmakologische Wirksamkeit dieser Substanzen findet man in der Literatur nicht.

Es ist bekannt, daß zahlreiche Erkrankungen der Haut unter anderem auch durch Verhornungsstörungen derselben verursacht werden, die durch eine vermehrte Bildung und/oder eine verminderte Abschilferung von Hornzellen zustande kommt.

So ist nach heutigem Wissen an der Entstehung der Acne vulgaris und verwandter Erkrankungen des Talgdrüsenfollikels, wie zum Beispiel der Acne estivalis und der Acne venenata neben einer erhöhten Sebumproduktion und einer Besiedelung des Follikellumens mit Propionibacterium acnes auch eine Verhornungsstörung des Follikelepithels beteiligt.

Ichthyosiforme Dermatosen und follikuläre Hyperkeratosen sind Verhornungsstörungen der Haut, Psoriasis, parapsoriatische Erkrankungen der Haut und lichinoide Hauterkrankungen sind ebenfalls gekennzeichnet durch eine Verhornungsstörung und zusätzlich durch entzündliche Prozesse, sowie eine vermehrte Durchblutung und Zellinfiltrate.

Bei Warzen und Dermatomykosen ist die Verhornungsstörung neben der mikrobiologischen Komponente für den Krankheitsverlauf von Bedeutung.

Derartige mit Verhornungsstörungen verbundenen Erkrankungen der Haut werden nach dem bekannten Stand der Technik mit Keratinolytika, wie Harnstoff, Salicylsäure oder Benzoylperoxid mit Glucocortikoiden, Retinoiden und im Falle der Aknetherapie mit lokal oder systemisch verabreichten Antibiotika behandelt. Der Nutzen dieser Therapeutika ist entweder aufgrund ihrer begrenzten Wirksamkeit, wie bei den Keratolytika, oder wegen der lokaltoxischen sowie systemischen Nebenwirkungen bis hin zur Teratogenität der Retinoide, eingeschränkt. Aus diesen Grunde sind die Therapiemöglichkeiten von Erkrankungen, die mit Verhornungsstörungen der Haut einhergehen, bislang unbefriedigend.

Es wurde nun gefunden, daß die Hydroxyalkancarbonsäure-Derivate der allgemeinen Formel I gemäß Patentanspruch 1 überraschenderweise eine ausgeprägte antiproliferative Wirkung auf Keratinocyten besitzen, so daß zu erwarten ist, daß Arzneimittel, die diese Wirkstoffe enthalten, zur lokalen Behandlung von mit Verhornungsstörungen und/oder Hyperproliferation der Epidermis verbundenen Erkrankungen der Haut sehr geeignet sind. Diese Hydroxyalkancarbonsäure-Derivate zeigen weiterhin eine ausgeprägte komedolytische, antiinflammatorische und antimikrobielle Wirksamkeit und haben den Vorzug einer sehr geringen Toxizität, so daß diese Wirkstoffe enthaltende Arzneimittel zur lokalen Behandlung zahlreicher Erkrankungen der Haut und Schleimhaut Anwendung finden können.

Derartige Erkrankungen sind beispielsweise:

a) Erkrankungen der Talgdrüsen und verwandte Erkrankungen wie die Akne vulgaris, die Akne venenata, Sonderformen der endogenen Akne (wie zum Beispiel die Akne neonatorum, die Akne infantum, die Akne aestivalis, die Akne excoriee und die gramnegative Folliculitis), die Rosacea, die periorale Dermatitis, die seborrhoische Dermatitis und das seborrhoische Ekzem.

b) Verhornungsstörungen der Haut wie zum Beispiel die ichthyosiformen Dermatosen (wie die Ichthyosis vulgaris, die geschlechtsgebunden vererbte Ichthyosis, die lamelläre Ichthyosis, die epidermolytische Ichthyosis und das Conradi Syndrom), folliculäre Hyperkeratosen (wie die Keratosis pilaris, die Keratosis

punctata, die Keratosis striata, die Keratosis senilis, die Pityriasis rubra pilaris und die Dyskeratosis follicularis vegetans (M. Darier) oder umschriebene Hyperkeratosen (wie palmare und plantare Hyperkeratosen und palmares und plantares Keratoderm).

c) Psoriasis und parapsoriatische Erkrankungen (wie die Parapsoriasis guttata, die Parapsoriasis variegata und die Parapsoriasis en plaques).

d) Lichenoide Dermatosen (wie Lichen ruber planus und der Lichen simplex chronicus).

e) Warzen (wie Verruca vulgaris, Verruca planae juvenilis und Plantarwarzen).

f) Dermatomycosen (wie Tinea pedis oder Tinea corporis).

g) Bakteriell superinfizierte Ekzeme und

h) prämaligne Erkrankungen der Epidermis, wie zum Beispiel oberflächliche aktinische Keratosen.

Die antiproliferative Wirkung auf Keratinocyten wurde in vitro wie folgt ermittelt:
Primärkulturen neonataler muriner Keratinocyten werden in serumfreien Medium 28 Stunden lang mit den Prüfsubstanzen und terminal 4 Stunden lang mit Methyl-3H Thymidin inkubiert.

Der 3H-Thymidineinbau in die DNA der Keratinocyten dient als Maß für die Proliferationsrate derselben. In diesem Test werden folgende Ergebnisse erzielt:

Tabelle 1

| Nr. | Testsubstanz | mol/l | % Hemmung* |
|---|---|---|---|
| I | 11-Hydroxy-undecansäuredimethylamid | $10^{-5}$<br>$10^{-4}$ | 30 %<br>70 % |
| II | 12-Hydroxy-dodecansäuredimethylamid | $10^{-5}$<br>$10^{-4}$ | 20 %<br>85 % |
| III | 13-Hydroxy-tridecansäure-dimethylamid | $10^{-5}$<br>$10^{-4}$ | 70 %<br>95 % |
| IV | 14-Hydroxy-tetradecansäure-dimethylamid | $10^{-5}$<br>$10^{-4}$ | 85 %<br>90 % |
| V | 13-Hydroxy-tridecansäuredipropylamid | $10^{-5}$<br>$10^{-4}$ | 91 %<br>92 % |
| VI | 13-Hydroxy-tridecansäuretetramethylenamid | $10^{-5}$<br>$10^{-4}$ | 24 %<br>92 % |
| VII | 13-Hydroxy-tridecansäure(2,4-dimethyl-3-oxa-pentamethylen)-amid | $10^{-5}$<br>$10^{-4}$ | 22 %<br>62 % |
| VIII | 13-Acetoxy-tridecansäuredimethylamid | $10^{-5}$<br>$10^{-4}$ | 32 %<br>89 % |
| IX | 13-(2,2-Dimethylpropionyloxy)-tridecansäure-dimethylamid | $10^{-5}$<br>$10^{-4}$ | 86 %<br>93 % |

* bezogen auf die Vehikel-Kontrolle

Die komedolytische Wirksamkeit der Hydroxyalkancarbonsäure-Derivate wird ermittelt, indem man an der Innenseite von Kaninchenohren durch topische Applikation von 200 µl Tetradecan nach einer 10tägigen Behandlung komedonenähnliche Veränderungen der Haarfollikel mit Epithelhyperplasie und Hornzellenretentien induziert und auf die so behandelten Stellen 11 Tage lang täglich jeweils 200 µl einer 5 %igen ethanolischen Lösung der Prüfsubstanz topisch appliziert.

Dann werden die behandelten Areale ausgestanzt und die Verkleinerung der erweiterten Follikellumina ermittelt, welche als Maß für die komedolytische Aktivität gilt.

In diesem Test zeigt eine 5 %ige Lösung von N,N-Dimethyl-13-hydroxy-tridecansäureamid eine 44,5 %ige Hemmung bezogen auf die Vehikelkontrolle.

Zur Bestimmung der antimikrobiellen Wirksamkeit werden unter den üblichen Bedingungen Suspensionskulturen von grampositiven Bakterien und Dermatophyton in Gegenwart von reihenverdünnten Prüfsubstanzen angelegt. Das bakterielle Wachstum wird anhand der Trübung der Kulturen verfolgt und es wird die minimale Hemmkonzentration (MHK) und die minimale biozide Konzentration (MBK) ermittelt. Tabelle 2

zeigt die in diesem Test ermittelten Ergebnisse:

| Testsubstanz | Staphylococcus aureus µg/l | | Staphylococcus epidermidis µg/l | | Streptococcus pyogenes µg/l | | Propionibakterium acnes µg/l | | Bacillus subtilis µg/l | |
|---|---|---|---|---|---|---|---|---|---|---|
| | MHK | MBK | MHK | MBK | MHK | MBK | MHK | MBK | MHK | MBK |
| I | >0,085 | >0,340 | >0,170 | 0,680 | 0,042 | 0,085 | >0,170 | >0,678 | >0,085 | >0,340 |
| II | 0,040 | 0,161 | 0,161 | 0,322 | 0,020 | 0,040 | 0,161 | 0,644 | 0,040 | 0,161 |
| III | >0,076 | >0,304 | >0,152 | >0,304 | 0,038 | 0,076 | >0,152 | >0,607 | >0,076 | >0,304 |
| IV | 0,098 | 0,196 | >0,196 | 0,392 | 0,025 | 0,049 | 0,196 | 0,784 | 0,049 | 0,196 |
| V | 0,053 | 0,106 | 0,214 | 0,214 | 0,013 | 0,013 | 0,106 | 0,427 | 0,053 | 0,214 |

| Testsubstanz | Trichophyton rubrum µg/l | | Trichophyton mentagrophytes µg/l | | Microsporum canis µg/l | | Aspergillus niger µg/l | |
|---|---|---|---|---|---|---|---|---|
| | MHK | MBK | MHK | MBK | MHK | MBK | MHK | MBK |
| I | 0,085 | 0,340 | 0,085 | 0,340 | 0,170 | 0,340 | 0,170 | 0,680 |
| II | 0,080 | 0,322 | 0,080 | 0,322 | 0,080 | 0,322 | 0,161 | 0,644 |
| III | 0,076 | 0,304 | 0,076 | 0,304 | 0,076 | 0,304 | 0,304 | 0,608 |
| IV | 0,098 | 0,392 | 0,098 | 0,392 | 0,098 | 0,392 | >0,186 | >0,784 |
| V | 0,106 | 0,427 | 0,106 | 0,427 | 0,106 | 0,427 | 0,214 | 0,427 |

I = 14-Hydroxy-tetradecansäure-dimthylamid
II = 13-Hydroxy-tridecansäure-dimethylamid
III = 12-Hydroxy-dodecansäure-dimethylamid
IV = 13-Hydroxy-tridecansäure-dipropylamid
V = 13-(2,2-Dimethylpropionyloxy)-tridecansäure-dimethylamid

Die antiinflammatorische Wirksamkeit der Hydroxyalkancarbonsäure-Derivate kann mittels des Rattenohrtests ermittelt werden, der wie folgt durchgeführt wird.

An der Außenseite von Rattenohren wird durch topische Applikation von 50 µl einer 5 %igen Lösung von Crotonöl in Ethanol ein Odem induziert. Gleichzeitig wird eine 5 %ige Lösung der Prüfsubstanzen in Ethanol ebenfalls topisch appliziert 5 Stunden nach der Applikation wird die Hemmung der Gewichtszunahme des behandelten Ohres ermittelt, welche ein Maß für die antiinflammatorische Wirksamkeit der Verbindungen ist. Tabelle 3 zeigt die in diesen Test erhaltenen Ergebnisse.

Tabelle 3

| Nr. | Substanz | % Hemmung* |
|---|---|---|
| I | 11-Hydroxy-undecansäure-dimethylamid | 54 |
| II | 12-Hydroxy-dodecansäure-dimethylamid | 31 |
| III | 13-Hydroxy-tridecansäure-dimethylamid | 64 |
| IV | 14-Hydroxy-tetradecansäure-dimethylamid | 55 |

* bezogen auf die Vehikel-Kontrolle

Zur Ermittlung der akuten Toxizität der erfindungsgemäßen Substanzen werden weißen Mäusen 100 mg/kg 13-Hydroxy-tridecansäure-dimethylamid intraperitoneal appliziert. 6 Stunden, 24 Stunden und 7 Tage

nach der Applikation hatte sich der Allgemeinzustand und das Differentialblutbild der Versuchstiere nicht auffällig verändert.

Im Rahmen der bislang durchgeführten pharmakologischen Untersuchungen wurden bislang überwiegend solche Hydroxyalkancarbonsäure-Derivate der allgemeinen Formel I untersucht, die eine freie Hydroxygruppe tragen. Es ist aber sehr wahrscheinlich, daß alle Verbindungen mit einer veresterten Hydroxygruppe in vivo das gleiche Wirkungsspektrum zeigen werden, da diese Ester sehr wahrscheinlich bei der Penetration durch die Haut hydrolytisch gespalten werden. Die Hydrolysegeschwindigkeit der Ester und die Lipophilität dieser Verbindungen (und somit deren Penetrationsfähigkeit) wird bei den verschiedenen Estern unterschiedlich sein, wodurch es möglich sein wird, die Wirkungsintensität und Wirkungsdauer der Hydroxyalkancarbonsäure-Derivate gezielt zu verändern.

Als Acylgruppen der Acyloxyalkancarbonsäure-Derivate der allgemeinen Formel I gemäß Patentanspruch 1 eignen sich solche, die 1 bis 16, - vorzugsweise 1 bis 8 Kohlenstoffatome - besitzen.

Solche Acylgruppen sind beispielsweise geradkettige oder verzweigte Alkanoylgruppen, Cycloalkylcarbonylgruppen, Cycloalkylalkanoylgruppen, Benzoylgruppen, Alkylbenzoylgruppen und Phenylalkanoylgruppen die in üblicher Weise beispielsweise durch Hydroxygruppen, Alkoxygruppen (Methoxygruppen etc.) Alkanoyloxygruppen (Acetoxygruppen etc.), Aminogruppen, Halogenatome oder Carboxylgruppen substituiert sein können. Als geeignete Acylgruppen seien beispielsweise genannt: Die Formylgruppe, die Acetylgruppe, die Propionylgruppe, die Butyrylgruppe, die 2-Methylpropionylgruppe, die Pentanoylgruppe, die 2,2-Dimethylpropionylgruppe, die Hexanoylgruppe, die Cyclopentylcarbonylgruppe, die 3-Cyclopropylpropionylgruppe, die Octanoylgruppe, die Dodecanoylgruppe, die Bezoylgruppe, die Hydroxyacetylgruppe, die Methoxyacetylgruppe, die Acetoxyacetylgruppe, die Aminoacetylgruppe, die 3-Carboxypropionylgruppe, die 2-Hydroxybenzoylgruppe, die 2-Acetoxybenzoylgruppe, die 4-Methoxyacetylgruppe, die 4-Chlorbenzoylgruppe, die 4-Methylbenzoylgruppe und die Phenylacetylgruppe.

Aufgrund der bisher durchgeführten Untersuchungen und allgemeiner Erfahrungsgrundsätze kann man davon ausgehen, daß nicht nur die bereits getesteten Hydroxyalkancarbonsäure-Derivate und deren Ester das aufgezeigte Wirkungsspektrum besitzen, sondern daß auch Strukturanaloge dieser Substanzen eine vergleichbare Wirksamkeit zeigen. Derartige Strukturanaloge sind Hydroxyalkancarbonsäure-Derivate der allgemeinen Formel I gemäß Patentanspruch 1 mit n in der Bedeutung von 7,8, 17,18 oder insbesondere 9 und 10, 15 und 16, Hydroxyalkancarbonsäure-Derivate der allgemeinen Formel I gemäß Patentanspruch 1 mit $R_2$ und/oder $R_3$ (die unterschiedlich sein können) in der Bedeutung einer Alkylgruppe mit 2 bis 8 (vorzugsweise 2 bis 4) Kohlenstoffatomen, wie die Ethylgruppe, Propylgruppe, 2-Propylgruppe sowie Hydroxyalkancarbonsäure-Derivate der allgemeinen Formel I gemäß Patentanspruch 1 mit $R_2$ und $R_3$ in der Bedeutung einer gegebenenfalls durch ein Sauerstoffatom oder ein Stickstoffatom unterbrochene Alkylengruppe mit 4 bis 8 Kohlenstoffatomen. Derartige Alkylengruppen sind vorzugsweise Gruppierungen, welche gemeinsam mit dem Amidstickstoff ein Ringsystem der Teilformel VI bilden:

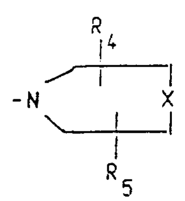 (VI),

worin

$R_4$ und $R_5$ unabhängig voneinander ein Wasserstoffatom oder eine Methylgruppe darstellen und

X eine Kohlenstoff-Kohlenstoffbindung, eine Methylengruppe, ein Sauerstoffatom, eine NH-Gruppe oder eine $>N-CH_3$ Gruppe symbolisiert, bilden.

Derartige Ringsysteme sind beispielsweise der Pyrrolidinring, der Piperidinring, der Piperazinring und der Morpholinring sowie deren Methyl- oder Dimethylsubstituenten tragende Derivate.

Die neuen Hydroxyalkancarbonsäure-Derivate der allgemeinen Formel I gemäß Patentanspruch 1 können beispielsweise nach dem in Patentanspruch 11 gekennzeichneten Verfahren hergestellt werden, welches unter Bedingungen durchgeführt werden kann, die dem Fachmann wohl bekannt sind.

Das erfindungsgemäße Verfahren gemäß Verfahrensvariante 11a wird unter Bedingungen durchgeführt, die man üblicherweise zur Decarboxylierung von Malonsäure-Derivaten verwendet. Die Reaktion erfolgt durch Erhitzen der Verbindungen der Formel II auf 80° C-180° C, wobei die Decarboxylierung in Abwesenheit eines Lösungsmittels oder in Anwesenheit eines hochsiedenden Lösungsmittels (wie Xylol, Chlorbenzol oder Decalin) durchgeführt werden kann.

Die für diese Verfahrensvariante benötigten Ausgangsverbindungen der allgemeinen Formel II können beispielsweise auf folgenden Wege synthetisiert werden:

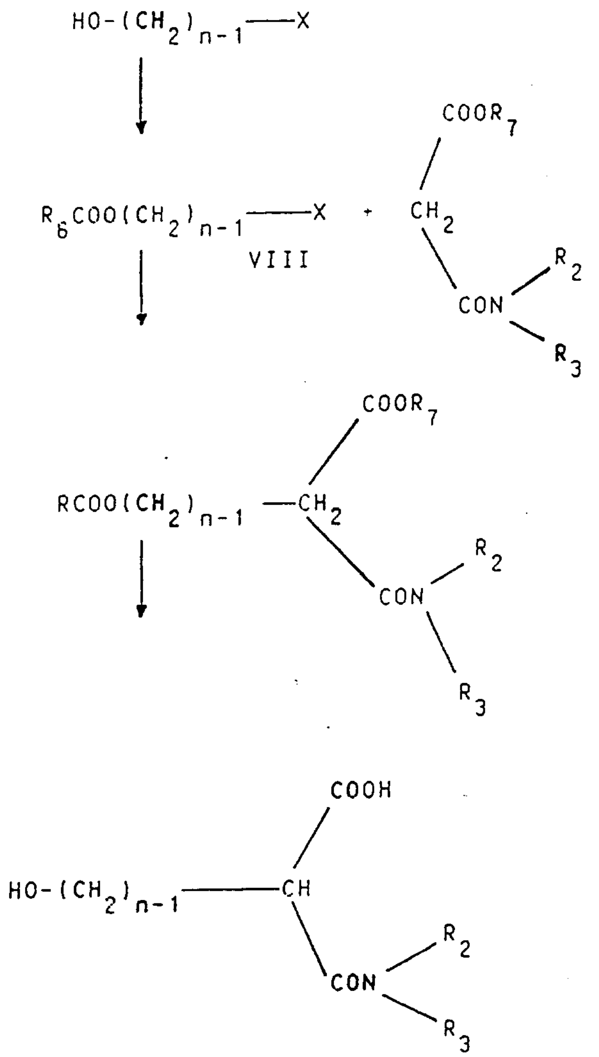

(VII),

VIII

(IX),

(X),

(II),

(In diesen Formeln hat n, $R_2$ und $R_3$ die obengenannte Bedeutung, X stellt ein Halogenatom - Chlor, Brom oder Jod - dar und $R_6$ sowie $R_7$ sind niedere Alkylgruppen mit bis zu 4 Kohlenstoffatomen, vorzugsweise Methylgruppen oder Ethylgruppen).

Die als Ausgangsverbindungen verwendeten ω-Halogenalkanole der Formel VII werden beispielsweise mit Acetanhydrid verestert und dann unter den üblichen Bedingungen der Malonesterkondensation mit dem Malonsäure-Derivat der allgemeinen Formel IX umgesetzt (beispielsweise, indem man das Malonsäure-Derivat in einem aprotischen Lösungsmittel wie Dimethylformamid mittels Natriumhydrid in das Enolat überführt und dieses mit der Halogenverbindung VIII umsetzt). Die erhaltene Verbindung der Formel X kann dann - beispielsweise mittels Basen - hydrolytisch gespalten werden und man erhält die Verbindungen der Formel II.

Das erfindungsgemäße Verfahren gemäß Verfahrensvariante 11b erfolgt ebenfalls unter an sich bekannten Bedingungen. Es kann beispielsweise unter den Reaktionsbedingungen durchgeführt werden, wie sie in der bereits erwähnten Publikation von G. Czichocki et. al angewendet werden.

Das erfindungsgemäße Verfahren gemäß Verfahrensvariante 11c wird unter Bedingungen durchgeführt, die man üblicherweise bei der reduktiven Umwandlung von Alkoxycarbonylgruppen zu Hydroxymethylgruppen anwendet. So ist es grundsätzlich möglich, diese Reaktion mit katalytisch aktiviertem Wasserstoff durchzuführen, jedoch besteht hier die Gefahr, daß auch die Amidgruppe zumindest teilweise ebenfalls reduziert wird. Sehr gute Ergebnisse erzielt man bei der Reduktion mit komplexen Metallhydriden, insbesondere von Natriumborhydrid in inerten Lösungsmitteln, wie tert.-Butanol und Methanol.

Die für diese Verfahrensvariante benötigten Ausgangsverbindungen können aus den entsprechenden Dicarbonsäurediestern der allgemeinen Formel XI wie folgt dargestellt werden:

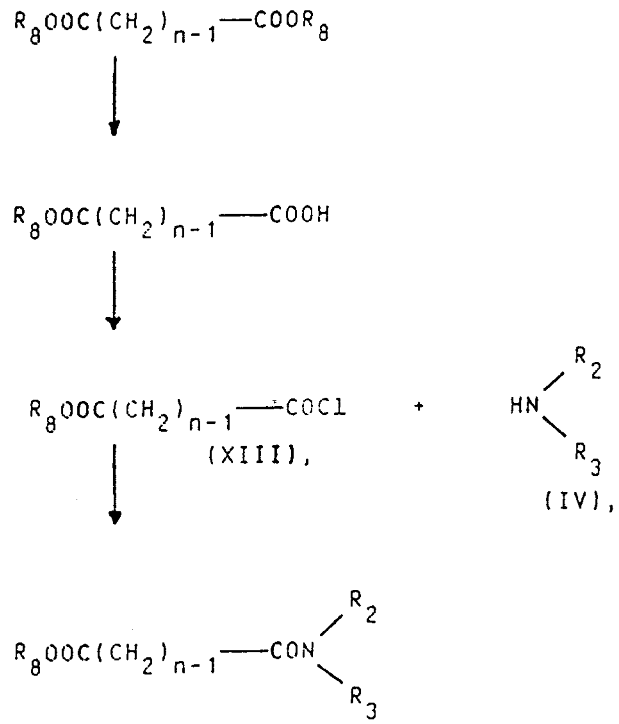

(XI),

(XII),

(XIII), (IV),

(V),

(In diesen Formeln hat n, $R_2$ und $R_3$ die oben genannte Bedeutung und $R_8$ stellt jeweils eine niedere Alkylgruppe mit maximal 4 Kohlenstoffatomen - insbesondere die Methylgruppe oder die Ethylgruppe - dar.)

Die Dicarbonsäurediester der Formel XI werden mittels Basen wie zum Beispiel Bariumhydroxid partiell zu den Dicarbonsäure-monoestern der Formal XII hydrolysiert. Diese werden beispielsweise mittels Thionylchlorid in die Säurechloride der Formel XIII überführt, welche ihrerseits mit den Aminen der allgemeinen Formel IV umgesetzt werden.

Es wurde bereits eingangs ausführlich dargelegt, daß ein Aspekt der vorliegenden Erfindung die Verwendung der Hydroxyalkancarbonsäure-Derivate der allgemeinen Formel I zur Herstellung eines Arzneimittels für die lokale Behandlung von Erkrankungen der Haut und Schleimhaut ist.

Die Herstellung derartiger topisch zu applizierbaren Arzneimittel ist an sich vorbekannt. Andererseits ist es aber auch möglich, neue, den speziellen Bedürfnissen der Haut angepaßte Zubereitungen zu erstellen.

Die Herstellung solcher topischen Zubereitungen erfolgt in üblicher Weise, indem man die Wirkstoffe mit geeigneten Zusätzen in die gewünschte Applikationsform, wie zum Beispiel eine Lösung, eine Milch, eine Lotion, eine Creme, eine Salbe, oder eine Paste überführt, In der so formulierten Zubereitung ist die Wirkstoffkonzentration von der Applikationsform abhängig. Vorzugsweise wird eine Wirkstoffkonzentration von 5 bis 30 Gewichtsprozent verwendet.

Die Milch, Lotion oder Creme (Öl/Wasser-Emulsionen) und die Salbe (Wasser/Öl-Emulsionen__ kann in konventioneller Weise unter Verwendung konventioneller Emulgatoren hergestellt werden (Kirk Othmer: Enzyclopedia of Chemical Technology, 3. Auflage, 1979; John Wiley & Sons, New York, Vol. 8, Seiten 900 - 930, und Dr. Otto-Albrecht Neumüller: Römpps Chemie Lexikon, 7. Auflage, 1973; Franckh'sche Verlagshandlung Stuttgart, Seiten 1009 - 1013). Die für diese Emulsionen verwendeten Wachse, Emulgatoren und übrigen Zusätze sind die gleichen, wie man sie konventioneller Weise verwendet (Dr. Otto-Albrecht Neumüller: Römpps Chemie Lexikon, 7, Auflage, 1973; Franckh'sche Verlagshandlung Stuttgart, Seiten 1427 und 1428).

Die erfindungsgemäße topische Zubereitung kann aus einem oder zwei Wirkstoffen hydrophilen und/oder lipophilen Zusätzen, Fettphase, Öl/Wasser-Emulgator, wäßriger Phase und Konservierungsmittel bestehen.

Als hydrophile und/oder lipophile Zusätze können Feuchthaltefaktoren (Hydrokomplexe), wie zum Beispiel Propylenglykol, Glycerin, Polyäthylenglykole, Vitalkomplexe (wie zum Beispiel Placentaextrakte),

Enzyme, Kräuterauszüge (wie zum Beispiel Hammamelis Extrakt oder Kamillenextrakt) oder Proteine (wie zum Beispiel Collagen) eingesetzt werden, Als ölige Phase oder als Fettphase in der Öl/Wasser-Emulsion eignen sich Kohlenwasserstoffe, wie zum Beispiel Squalen, Vaseline, Paraffine oder Stearin, oder Wachse, wie zum Beispiel Bienenwachs oder tierische oder pflanzliche Öle, wie Olivenöl, Erdnußöl, feines Knochenöl, Mandelöl, Jojoba-Öl, Lanolin oder Sonnenblumenöl. Geeignete Öl/Wasser-Emulgatoren sind beispielsweise Stearylalkohol, Polyoxyäthylenstearate (wie zum Beispiel MYRJ®), Komplexemulgatoren (wie zum Beispiel Amphoterin®) und Sorbitanfettsäureester (wie zum Beispiel Tween 80®), Carboxyvinylpolymerisate (wie zum Beispiel Carbopol®), Fettalkohole wie zum Beispiel Cetylalkohol, Myristylalkohol oder Mischester (wie zum Beispiel Dehymuls®). Die wäßrige Phase kann zusätzlich noch Puffersubstanzen, wie zum Beispiel das Dinatriumsalz der Äthylendiamin-N,N,N',N'-tetraessigsäure und Konservierungsmittel, wie Benzoesäure, Chlorquinaldol, Parabene oder Benzalkoniumchlorid, enthalten.

Die Emulsion wird zusätzlich mit einem oder zwei Wirkstoff(en) und gegebenenfalls noch mit Duftstoffen, wie zum Beispiel diejenige der Crematest®-Serie, versetzt und bis zur gleichmäßigen Verteilung derselben gerührt.

Es ist oft vorteilhaft, den erfindungsgemäßen Mitteln zusätzlich noch ca. 1 bis 4 Gewichtsprozent einer keratolytisch wirksamen Substanz, wie zum Beispiel Salicylsäure oder Resorcin - bezogen auf das Gesamtgewicht des Mittels - zuzusetzen.

Die nachfolgenden Ausführungsbeispiele dienen zur näheren Erläuterung der Erfindung.

## Beispiel 1

a) 25 g 11-Brom-undecanol werden in 200 ml Acetanhydrid 5 Stunden lang unter Rückfluß erhitzt. Dann destilliert man das überschüssige Acetanhydrid im Vakuum ab, fraktioniert den Rückstand im Hochvakuum und erhält 28,6 g 1-Acetoxy-11-bromundecan vom Siedepunkt 118-120° C bei 6,7 Pa.

b) Eine Suspension von 0,6 g Natriumhydrid und 3 g Malonsäure-ethylester-dimethylamid in 30 ml Dimethylformamid wird eine Stunde lang bei 22° C gerührt, mit 6,9 g 1-Acetoxy-11-bromundecan versetzt und 16 Stunden lang bei 50° C gerührt. Anschließend rührt man die Reaktionsmischung in 200 ml Eiswasser, extrahiert mit Essigsäureethylester, trocknet die organische Phase über Natriumsulfat und engt sie im Vakuum ein. Man erhält so 5,3 g 11-Acetoxyundecan-malonsäureethylester-dimethylamid als hellgelbes Ol.

c) 4,04 g des so erhaltenen Produkts werden in 21,8 ml 1n wässriger Natronlauge suspendiert und 5 Stunden lang unter Rückfluß erhitzt. Man läßt die Reaktionsmischung erkalten, säuert sie mit 10n wässriger Salzsäure an, saugt das abgeschiedene Produkt ab und erhält so 3,5 g 1-Hydroxyundecanmalonsäure-dimethylamid als Rohprodukt.

d) Das erhaltene Rohprodukt wird bis zur Beendigung der $CO_2$ Abspaltung auf 150° C erhitzt (ca. eine Stunde), dann läßt man die Mischung erkalten und kristallisiert das erhaltene Rohprodukt aus Ethylacetat um. Man erhält so 1,92 g 13-Hydroxy-tridecansäure-dimethylamid vom Schmelzpunkt 69-71° C.

## Beispiel 2

a) 200 g Tridecan-1,13-disäure-dimethylester werden in 200 ml Methanol gelöst, mit einer Lösung von 116 g Bariumhydroxid-Octahydrat in 1,5 l Methanol versetzt und 38 Stunden lang bei 22° C gerührt. Dann saugt man den Niederschlag ab, suspendiert ihn in 3 Liter Eis-Wasser und säuert ihn mit 75 ml 10n wässriger Salzsäure an. Man extrahiert mit Essigsäureethylester, wäscht die organische Phase mit Wasser, trocknet sie über Natriumsulfat, engt sie im Vakuum ein und erhält 179 g Tridecan-1,13-disäure-monomethylester vom Schmelzpunkt 50-51° C.

b) Man gibt 26,3 ml Thionylchlorid zu 62,4 g Tridecan-1,13-disäure-monomethylester, erhitzt 2 Stunden lang auf 80° C und engt die Reaktionsmischung im Vakuum ein. Der Rückstand wird in 50 ml Diethylether gelöst und innerhalb von 10 Minuten unter Eiskühlung zu einer Suspension von 67,3 ml einer 40 %igen wässrigen Dimethylaminlösung und 65 ml Diethylether zugetropft. Man rührt die Mischung in Eiswasser, extrahiert mit Essigsäureethylester, wäscht die organische Phase mit Wasser, trocknet sie über Natriumsulfat und engt sie im Vakuum ein. Das erhaltene Rohprodukt wird aus Hexan umkristallisiert und man erhält 38,3 g Tridecan-1,13-disäure-methylester-dimethylamid vom Schmelzpunkt 39-40° C.

c) 10 g Tridecan-1,13-disäure-methylester-dimethylamid werden in 140 ml tert.-Butanol gelöst, mit 13,1 g Natriumborhydrid versetzt und auf 80° C erhitzt. Innerhalb von 45 Minuten werden 28 ml Methanol bei 80° C zugetropft. Dann läßt man die Mischung erkalten, versetzt sie mit 350 ml Eiswasser und extrahiert mit Essigsäureethylester, wäscht die organische Phase, trocknet sie über Natriumsulfat und engt sie im

Vakuum ein. Das erhaltene Rohprodukt wird aus Cyclohexan-Essigsäureethylester umkristallisiert und man erhält 6,4 g 13-Hydroxytridecansäure-dimethylamid vom Schmelzpunkt 68-70° C.

Beispiel 3

Unter den Bedingungen des Beispiels 2, aber ausgehend von dem Undecan-1,11-disäure-dimethylester wird das 11-Hydroxyundecansäure-dimethylamid hergestellt.

Beispiel 4

Unter den Bedingungen des Beispiels 2, aber ausgehend von dem Dodecan-1,12-disäure-dimethylester wird das 12-Hydroxydodecansäure-dimethylamid hergestellt.

Beispiel 5

Unter den Bedingungen des Beispiels 2, aber ausgehend von dem Tetradecan-1,14-disäure-dimethylester wird das 14-Hydroxy-tetradecansäure-dimethylamid hergestellt.

Beispiel 6

15,0 g 13-Hydroxy-tridecansäure-dimethylamid werden mit 64,7 ml Essigsäureanhydrid und 80,4 ml Pyridin versetzt und 16 Stunden lang bei Raumtemperatur gerührt. Dann gießt man die Mischung auf Eiswasser, extrahiert mit Essigsäureethylester, wäscht die organische Phase, trocknet sie über Natriumsulfat und engt sie im Vakuum ein. Das gebildete Rohprodukt wird aus Hexan umkristallisiert und man erhält 13,27 g 13-Acetoxy-tridecansäure-dimethylamid vom Schmelzpunkt 44-45,5° C.

Beispiel 7

Zu einer Mischung von 15,0 g 13-Hydroxytridecansäure-dimethylamid und 80 ml Pyridin werden unter Eiskühlung 84,2 ml 2,2-Dimethylpropionsäurechlorid getropft. Man rührt die Reaktionsmischung 14 Stunden lang bei Raumtemperatur und gießt sie auf Eiswasser. Nach Zugabe von 70 g Natriumhydrogencarbonat extrahiert man mit Essigsäureethylester, wäscht die organische Phase, trocknet sie über Natriumsulfat und engt sie im Vakuum ein. Das erhaltene Rohprodukt wird über eine Kieselgelsäule (8x30 cm) mit Essigsäureethylester/Hexan (1+1) chromatographiert und man erhält 13,6 g 13-(2,2-Dimethylpropionyloxy)-tridecansäure-dimethylamid vom Schmelzpunkt 22° C.

Beispiel 8

a) 176,1 g Tridecan-1,13-disäuremonomethylester werden mit 74,2 ml Thionylchlorid und 5,6 ml Dimethylformamid versetzt und 90 Minuten lang bei 80° C gerührt. Dann engt man die Mischung im Vakuum ein und erhält 191 g kristallinen Tridecan-1,13-disäurechlorid-methylester als Rohprodukt.

b) Zu einer Lösung von 25,0 g Tridecan-1,13-disäurechlorid-methylester-Rohprodukt in 100 ml Diethylether tropft man bei -10° C eine Lösung von 27,21 ml Dipropylamin in 25 ml Diethylether. Dann setzt man der Mischung noch 60 ml Diethylether zu und rührt sie noch 150 Minuten lang bei 0° C. Die Reaktionsmischung wird in ein Gemisch von Eis/verdünnter Salzsäure gegossen und mit Diethylether extrahiert. Die organische Phase wird gewaschen, über Natriumsulfat getrocknet, im Vakuum eingeengt und man erhält 29,34 g Tridecan-1,13-disäure-methylester-dipropylamid als Rohprodukt.

c) Zu einer siedenen Suspension von 29,34 g Tridecan-1,13-disäure-methylesterdipropylamid in 400 ml tert.-Butanol und 32,2 g Natriumborhydrid tropft man innerhalb von 45 Minuten unter Inertgas 68,8 ml Methanol. Dann erhitzt man die Reaktionsmischung noch 100 Minuten lang unter Rückfluß, läßt sie erkalten und gießt sie in eine Mischung aus Eis, Wasser und Natriumchlorid. Man extrahiert mit Essigsäureethylester, wäscht die organische Phase mit gesättigter wässriger Natriumchlorid-Lösung, trocknet sie über Natriumsulfat und engt sie im Vakuum ein. Der Rückstand wird über eine Kieselgelsäule (7x35 cm) mit Essigsäureethylester/Hexan (1+1) chromatographiert und man erhält 14,3 g 13-Hydroxytridecansäure-dipropylamid vom Schmelzpunkt 33,5-35,0° C.

Beispiel 9

a) Unter den Bedingungen des Beispiels 8b werden 25,0 g Tridecan-1,13-disäurechlorid-methylester mit 16,6 ml Pyrrolidin umgesetzt, aufbereitet und man erhält 24,32 g Tridecan-1,13-disäure-methylester-tetramethylenamid vom Schmelzpunkt 41-42° C.
b) In eine siedende Suspension von 24,32 g Tridecan-1,13-disäure-methylestertetramethylenamid in 350 ml tert.-Butanol und 29,28 g Natriumborhydrid werden innerhalb von 30 Minuten 62,6 ml Methanol eingetropft. Man erhitzt die Mischung noch 150 Minuten lang unter Rückfluß und arbeitet sie auf wie in Beispiel 8c beschrieben. Das so erhaltene Rohprodukt wird aus Cyclohexan/Essigsäureethylester umkristallisiert und ergibt 13,53 g 13-Hydroxytridecansäure-tetramethylenamid vom Schmelzpunkt 60-61° C.

Beispiel 10

a) Unter den Bedingungen des Beispiels 8b werden 25,0 g Tridecan-1,13-disäurechlorid-methylester mit 15,8 ml 2,6-Dimethylmorpholin umgesetzt und aufbereitet. Das erhaltene Roprodukt wird über eine Kieselgelsäule (5x100 cm) mittels Hexan-Essigsäureethylester/Hexan (1+2) Gradienten chromatographiert und man erhält 10,4 g Tridecan-1,13-disäure-methylester-(2,4-dimethyl-3-oxapentamethylen)amid als Ol.
b) Zu einer siedenden Suspension von 10,2 g Tridecan-1,13-disäure-methylester-(2,4-dimethyl-3-oxapentamethylen)amid in 150 ml tert.-Butanol und 12,28 g Natriumborhydrid wurden innerhalb von 20 Minuten 26,27 ml Methanol zugetropft. Dann erhitzt man die Mischung noch 150 Minuten lang unter Rückfluß, arbeitet sie auf wie in Beispiel 8c beschrieben, kristallisiert das erhaltene Rohprodukt aus Cyclohexan/Essigsäureethylester um und erhält 5,58 g 13-Hydroxy-tridecansäure-(2,4-dimethyl-3-oxapentamethylen)-amid vom Schmelzpunkt 86-87,5° C.

B) Ausführungsbeispiel betreffend eine galenische Zubereitung

Zusammensetzung

| | Gewichtsprozent |
|---|---|
| 13-Hydroxy-tridecansäure-dimethylamid | 20,0 |
| Benzoesäure | 0,1 |
| Salicylsäure | 2,0 |
| Glycerinmonostearat | 2,0 |
| Cetylalkohol | 3,0 |
| Polyoxyethylen(20)sorbitanmonooleat | 5,0 |
| Natrium-laurylethersulfat | 10,0 |
| Ethanolamin-laurylethersulfat | 1,0 |
| Olivenöl | 2,0 |
| Ascorbinsäure | 1,0 |
| Bidestilliertes Wasser | 53,9 |

Zubereitung

13-Hydroxy-tridecansäure-dimethylamid, Benzoesäure, Polyoxyethylen(20)sorbitanmonolat, Natrium-laurylethersulfat, Ethanolamin-laurylethersulfat und Wasser werden auf 60° C erwärmt und mit der Mischung von Salicylsäure, Glycerinmonostearat, Cetylalkohol, Olivenöl und Ascorbinsäure bei 40° C dispergiert.

**Patentansprüche**

**1.** Hydroxyalkancarbonsäure-Derivate der allgemeinen Formel I

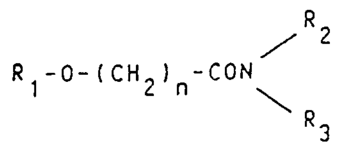 (I),

worin

| | |
|---|---|
| $R_1$ | ein Wasserstoffatom oder eine Acylgruppe mit maximal 16 Kohlenstoffatomen darstellt |
| $R_2$ und $R_3$ | jeweils Alkylgruppen mit maximal 8 Kohlenstoffatomen oder gemeinsam eine gegebenenfalls durch ein Sauerstoffatom oder ein Stickstoffatom unterbrochene Alkylengruppe mit 4 bis 8 Kohlenstoffatomen bedeutet und |
| n | die Ziffern 7 bis 18 symbolisiert mit der Maßgabe, daß es nicht die Ziffer 10 bedeutet, wenn $R_2$ und $R_3$ jeweils eine Ethylgruppe oder jeweils eine n-Butylgruppe darstellt und daß es nicht die Ziffer 15 bedeutet, wenn $R_2$ und $R_3$ jeweils eine Methylgruppe darstellt, |

**2.** 11-Hydroxy-undecansäure-diemethylamid.

**3.** 12-Hydroxy-dodecansäure-diemethylamid.

**4.** 13-Hydroxy-tridecansäure-dimethylamid.

**5.** 14-Hydroxy-tetracansäure-dimethylamid.

**6.** 13-Acetoxy-tridecansäure-dimethylamid.

**7.** 13-(2,2-Dimethylpropionyloxy)-tridecansäure-dimethylamid.

**8.** 13-Hydroxy-tridecansäure-dipropylamid.

**9.** 13-Hydroxy-tridecansäure-tetramethylenamid.

**10.** 13-Hydroxy-tridecansäure-(2,4-dimethyl-3-oxa-pentamethylen)-amid.

**11.** Verfahren zur Herstellung von Hydroxyalkancarbonsäure-Derivaten der allgemeinen Formel I gemäß Patentanspruch 1, dadurch gekennzeichnet,daß man

a) eine Verbindung der allgemeinen Formel II

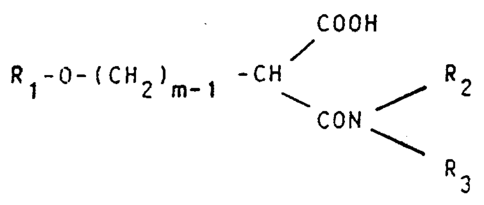 (II),

worin m, $R_1$, $R_2$ und $R_3$ die im Patentanspruch genannte Bedeutung besitzen, decarboxyliert, oder

b) eine Carbonsäure der allgemeinen Formel III

$R_1$-O-$(CH_2)_m$-COOH (III),

worin m und $R_1$ die oben genannte Bedeutung besitzen, mit einem Amin der allgemeinen Formel IV

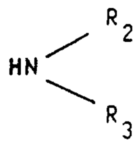

(IV),

worin $R_2$ und $R_3$ die obengenannte Bedeutung besitzen kondensiert, oder

c) eine Verbindung der allgemeinen Formel V

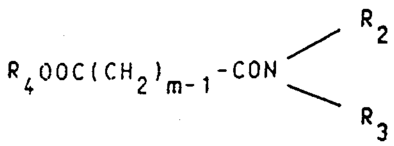

(V),

worin $R_2$ und $R_3$ die oben genannte Bedeutung besitzen und $R_4$ eine Alkylgruppe mit bis zu 6 Kohlenstoffatomen darstellt, mittels katalytisch aktiviertem Wasserstoff oder komplexen Metallhydriden reduziert und gewünschtenfalls die Ester der allgemeinen Formel I verseift und freie Alkohole der allgemeinen Formel I verestert.

Europäisches Patentamt

# EUROPÄISCHER RECHERCHENBERICHT

Nummer der Anmeldung
EP 94 25 0072

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | US-A-2 936 325 (REMES ET. AL.)<br>* Ansprüche 1-5 *<br>--- | 1 | C07C235/06<br>C07D295/18<br>A61K31/16<br>A61K31/40<br>A61K31/535 |
| X | US-A-2 372 797 (SEGESSEMANN ET. AL.)<br>* Ansprüche 1,3 *<br>--- | 1 | |
| X | CHEMICAL ABSTRACTS, vol. 104, no. 17, 28. April 1986, Columbus, Ohio, US; abstract no. 148126g,<br>A. P. TULLOCH 'Mass spectra of pyrrolidides of oxo, hydroxy and trimethylsilyloxy octadecanoic acids'<br>Seite 625 ;Spalte 1 ;<br>* Zusammenfassung *<br>& LIPIDS<br>Bd. 20, Nr. 10 , 1985<br>Seiten 652 - 63<br>& 11TH COLLECTIVE INDEX<br>Seite 59916CS<br>* Pyrrolidine, 2,2,3,4,5,5-, 1-(18-hydroxy-1-oxo-octadecyl)- *<br>--- | 1 | RECHERCHIERTE SACHGEBIETE (Int.Cl.5) |
| D,A | CHEMICAL ABSTRACTS, vol. 83, no. 14, 6. Oktober 1975, Columbus, Ohio, US; abstract no. 117596d,<br>G. CZICHOCKI ET. AL. 'Anion-active surfactants from 10-undecenoic acid'<br>Seite 207 ;Spalte 1 ;<br>* Zusammenfassung *<br>& TENSIDE DETERG.<br>Bd. 11, Nr. 6 , 1974<br>Seiten 298 - 305<br>& 9TH COLLECTIVE INDEX<br>Seite 39750CS<br>* Undecamide, N,n-dibutyl-11-hydroxy-; Undecamide, N,N-diethyl-11-hydroxy- *<br>---<br>-/-- | 1-11 | C07C<br>C07D |

Der vorliegende Recherchenbericht wurde für alle Patentansprüche erstellt

| Recherchenort | Abschlußdatum der Recherche | Prüfer |
|---|---|---|
| DEN HAAG | 13. Juni 1994 | Helps, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 94 25 0072

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,A | JOURNAL OF THE CHEMICAL SOCIETY, C<br>Dezember 1968 , LETCHWORTH GB<br>Seiten 2821 - 7<br>D. F. JONES ET. AL. 'Microbiological oxidation of long chain aliphatic compounds . Part IV. Alkane derivatives having polar terminal groups.'<br>* Seite 2826, Spalte 2, Zeile 22 - Zeile 27 *<br>----- | 1-11 | |
| | | | RECHERCHIERTE SACHGEBIETE (Int.Cl.5) |

Der vorliegende Recherchenbericht wurde für alle Patentansprüche erstellt

| Recherchenort | Abschlußdatum der Recherche | Prüfer |
|---|---|---|
| DEN HAAG | 13. Juni 1994 | Helps, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)